# NEW EUROPEAN PATENT SPECIFICATION

(11) **EP 0 639 563 B2**
(45) Date of publication and mention of the opposition decision: **30.09.2015**
(45) Mention of the grant of the patent: 12.01.2005
(21) Application number: 94305752.1
(22) Date of filing: 03.08.1994
(51) Int. Cl.: C07C 405/00, A61K 31/557, A61P 27/06

(54) **Use of fluprostenol isopropyl ester for the manufacture of a medicament for the treatment of glaucoma and ocular hypertension**
Verwendung von Fluprostenolisopropylester zur Herstellung eines Arzneimittels zur Behandlung von Glaukom und okularer Hypertension
Utilisation de l' ester isopropylique de fluprostenol pour la fabrication d'un médicament pour le traitement du glaucome et de l'hypertension oculaire

(30) Priority: 03.08.1993 US 101598
(43) Date of publication of application: 22.02.1995
(60) Divisional application: 04077870.6
(73) Proprietor: Alcon Research, Ltd., Fort Worth, TX 76134-2099 (US)
(72) Inventor: Klimko, Peter G., Forth Worth, Texas 76132 (US); Bishop, John E, APT. B Nashua, New Hampshire 03062 (US); Desantis Jr., Louis, Forth Worth, Texas 76109 (US); Sallee, Verney L., Burleson, Texas 76028 (US); Zinke, Paul W., Fort Worth, Texas 76133 (US)
(74) Representative: Gillard, Richard Edward

(56) References cited:
- EP-A- 0 364 417
- EP-A- 0 603 800
- WO-A-94/08585
- WO-A1-93/00329
- DE-A- 2 223 365
- DE-A- 2 523 676
- DE-A- 2 534 989
- J.LIPID.MEDIAT., vol. 6, no. 1-3, pages 545-553, XP002009581 DAVID F. WOODWARD ET AL.: "Intraocular pressure effects of slective prostanoid receptor agonists involve different receptor subtypes according to radioligand binding studies"
- EXP.EYE RES., vol. 55, no. sup1, page s91 XP002009582 WOODWARD D.F. ET AL.: "Definition of prostanoid receptor subtypes by radioligand binding and the effects of selective agonists on intraocular pressure"
- N. PFEIFFER: 'KLIN. MONATSBL. AUGENHEILKD', vol. 203, F. ENKE VERLAG, STUTTGART pages 1 - 9
- L.Z. BITO ET AL.: 'The ocular effects of prostaglandins and the therapeutic potential of a new PGF²x analog, PhXA41 (latanoprost), for glaucoma management' JOURNAL OF LIPID MEDIATORS vol. 6, no. 1-3, 1993, pages 535 - 543
- K.S. CRAWFORD ET AL.: 'Dose-Related Effects of Prostaglandin F²x Isopropylester on Intraocular PressureRefraction, and Pupil Diameter in Monkeys' INVESTIGATIVE OPHTHALMOLOGY & VISUAL SCIENCE vol. 32, no. 3, 03 March 1991, pages 510 - 519
- J. STJERNSCHANTZ ET AL.: 'Phenyl substituted prostaglandin analogs for glaucoma treatment' DRUGS OF THE FUTURE vol. 17, no. 8, 1992, pages 691 - 704
- J. VILLUMSEN ET AL.: 'Prostaglandin F²x-isopropylester eye drops: effect on intraocular pressure in open-angle glaucoma' BRITISH JOURNAL OF OPHTHALMOLOGY vol. 73, 1989, pages 975 - 979
- A. R. GENNARO ET AL.: 'Remington's Pharmaceutical Sciences', vol. 17, 1985 page 1559
- S. BUDAVARI ET AL.: 'Boric acid definition', vol. 11, 1989, MERCK & CO., INC., RAHWAY, N.J. pages 203 - 204
- 'Hunnius Pharmazeutisches', vol. 6, 1986, WALTER DE GRUYTER, BERLIN - NEW YORK pages 174 - 175
- R. BERKOW ET AL.: 'The Merck Manual', vol. 16, 1992, MERCK RESEARCH LABORATORIES, RAHWAY N.J. deel R. BERKOW ET AL.
- J. VILLUMSEN ET AL.: 'Prostaglandin F²x-isopropylester eye drops: effects in normal human eyes' BRITISH JOURNAL OF OPHTHALMOLOGY vol. 73, 1989, pages 419 - 426
- L.Z. BITO ET AL.: 'The ocular hypotensive effects and side effects of prostaglandins on the eyes ofexperimental animals', 1989, ALAN R. LISS, INC., NEW YORK pages 349 - 368
- A. ALM ET AL.: 'Effects of topically applied PGF2U and its isopropylester on normal and glaucomatous human eyes', 1989, ALAN R. LISS, INC., NEW YORK pages 447 - 458
- C.B. CAMRAS ET AL.: 'Reduction of intraocular pressure in normal and glaucomatous primate (Aotus trivirgatus) eyes by topically applied prostaglandin F²x' CURRENT EYE RESEARCH vol. 1, no. 4, 1989, pages 205 - 209
- L.Z. BITO ET AL.: 'Ophthalmology Clinics of North America', vol. 2, March 1989 pages 65 - 76
- 'Stedman's Medical Dictionary', vol. 27, 2000, LIPPINCOTT WILLIAMS & WILKINS, PHILADELPHIA page 848
- Declaration of Dr. David H. Sherman, dated 23.05.2013
- D. TUTSCH, DR. MED. ET AL.: 'Roche Lexikon Medizin', vol. 2, 1987, URBAN & SCHWARZENBERG, MÜNCHEN - WIEN - BALTIMORE page 821

## Description

### BACKGROUND OF THE INVENTION

The present invention relates to the treatment of glaucoma and ocular hypertension. In particular, the present invention relates to the use of fluprostenol isopropyl ester to treat glaucoma and ocutar hypertension.

Cloprostenol and fluprostenol, both known compounds, are synthetic analogues of PGF_{2α}, a naturally-occurring F-series prostaglandin (PG). Structures for PGF_{2α} (I), cloprostenol (II), and fluprostenol (III), are shown below:

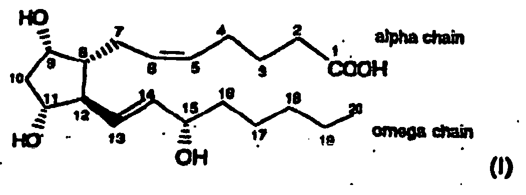

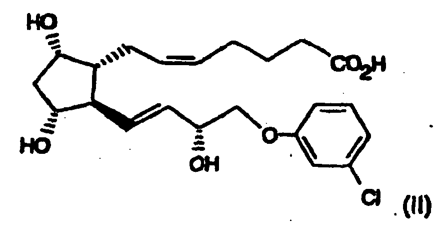

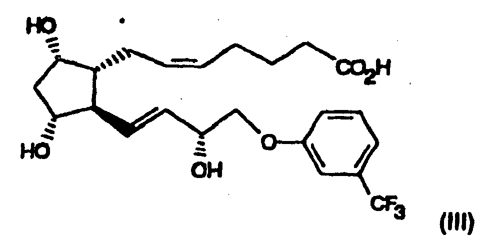

The chemical name for cloprostenol is 16-(3-chlorophenoxy)-17,18,19,20-tetranor PGF_{2α}. Monograph No. 2397 (page 375) of The Merck Index, 11th Edition (1989) is incorporated herein by reference to the extent that it describes the preparation and known pharmacological profiles of cloprostenol. Fluprostenol has the chemical name 16-(3-trifluoromethylphenoxy)-17,18,19,20-tetranor PGF_{2α}. Monograph No. 4121 (pages 656-657) of The Merck Index11th Edition (1989) is incorporated herein by reference to the extent that it describes the preparation and known pharmacological profiles of fluprostenol. Cloprostenol and fluprostenol are 16-aryloxy PGs and, in addition to the substituted aromatic ring, differ from the natural product, PGF_{2α} in that an oxygen atom is embedded within the lower (omega) chain. This oxygen interruption forms an ether functionality.

Naturally-occurring prostaglandins are known to lower intraocular pressure (IOP) after topical ocular instillation, but generally cause inflammation, as well as surface irritation characterized by conjunctival hyperemia and edema. Many synthetic prostaglandins have been observed to lower intraocular pressure, but such compounds also produce the aforementioned side effects. Various methods have been used in attempting to overcome the ocular side effects associated with prostaglandins. Stjernschantz et al. (EP 364 417 A1) have synthesized derivatives or analogues of naturally-occurring prostaglandins in order to design out selectively the undesired side effects while maintaining the IOP-lowering effect. Others, including Ueno et al. (EP 330 511 A2) and Wheeler (EP 435 682 A2) have tried complexing prostaglandins with various cyclodextrins.

The Stjernschantz et al. publication is of particular interest, as it demonstrates that certain synthetically-modified PGF_{2α} analogues retain the potent IOP-lowering effect of the parent (PGF_{2α} isopropyl ester) while decreasing the degree of conjunctival hyperemia. In this publication, the only modification to the PG structure is to the omega chain: the chain length is 4-13 carbon atoms "optionally interrupted by preferably not more than two heteroatoms (O, S, or N)" and includes a phenyl ring (substituted or unsubstituted) on the terminus (see page 3, line 44 to page 4, line 7). Stjernschantz et al. exemplify two subclasses within this definition:
(**1**) carbon-only omega chains, i.e., 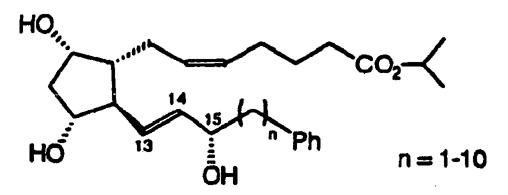
and (**2**) heteroatom-interrupted omega chains, i.e., 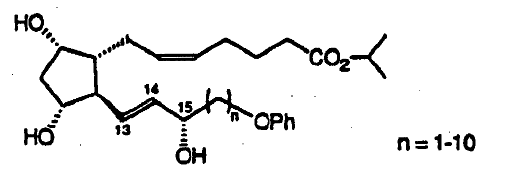 In particular, the 17-phenyl-18,19,20-trinor analogue of PGF_{2α} isopropyl ester (formula **1**, n=2) displayed a superior separation of toward and untoward activities. Furthermore, the 13,14-dihydro analogue of 17-phenyl-18,19,20-trinor PGF_{2α} isopropyl ester displayed an even more favorable separation of activities. Both 17-phenyl PGF_{2α} and its 13,14-dihydro congener fall into the former (formula **1**, carbon-only omega chain) subclass. Additional synthetic analogues employing the phenyl substituent on the end of the omega chain explored the effects of chain elongation, chain contraction, and substitution on the phenyl ring. However, such analogues showed no apparent therapeutic improvement over the preferred formulation, 13,14-dihydro-17-phenyl-18,19,20-trinor PGF_{2α} isopropyl ester.

Because they contain heteroatom (O) interruption of the omega chain, both cloprostenol and fluprostenol are generically included in the subclass defined in formula **2** by Stjernschantz et al. However, neither compound is specifically mentioned by Stjernschantz et al. and the disclosure is primarily related to carbon-only omega chains. The only example of a heteroatom-interrupted omega chain disclosed by Stjernschantz et al. is 16-phenoxy-17,18,19,20 tetranor PGF_{2α} isopropyl ester (see formula **2**, n=1). The IOP data revealed by Stjernschantz et al. for 16-phenoxy-17,18,19,20-tetranor PGF_{2α} isopropyl ester (see Stjernschantz et al, page 17, Table V) indicate an initial increase in IOP (1-2 hours after administration) followed by a decrease. Moreover, this compound displays unacceptable hyperemia (see Stjernschantz et al., Table IV, line 40). In short, data from Stjernschantz et al. demonstrate that the oxygen-interrupted omega chain subgeneric class of compounds (see formula **2**) displays an unacceptable therapeutic profile.

EP 0 603 800, which is prior art under Article 54(3) EPC, discloses combinations of prostaglandins of the F and E series. The F series prostaglandin has the formula: 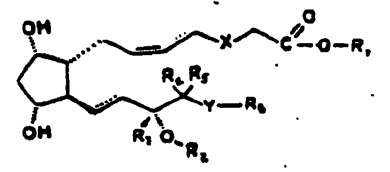 wherein:
- X and Y: can be the same or different, and are: CH₂ or O;
- R₁: is hydrogen, a cationic salt moiety, a pharmaceutically acceptable amine moiety or a pharmaceutically acceptable ester moiety derived from the corresponding alcohol; and
- R₂: is hydrogen or a pharmaceutically acceptable ester moiety derived from the corresponding carboxylic acid.
- R₃, R₄ and R₅: can be the same or different, and are: H or CH₃, with the proviso that if R₃ is CH₃, then R₄ and R₅ are H; and
- R₆: is: C₂₋₇ alkyl, thienyl or aryl, optionally substituted with one or more of the following: C₁₋₅ alkyl, trifluoromethyl, or a halogen;
with the proviso that if Y is O, and R₆ is aryl, then the aryl group must contain at least one substituent
and the E series prostglandins has the formula: 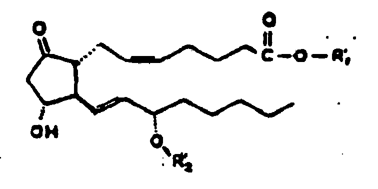 wherein:
R'₁ is hydrogen, a cationic salt moiety, a pharmaceutically acceptable amine moiety or a pharmaceutically acceptable ester moiety derived from the corresponding alcohol; and
R'₂ is hydrogen or a pharmaceutically acceptable ester moiety derived from the corresponding carboxylic acid.

WO 94/08585 (EP 0 664 707), which is also prior art under Article **54(3)** EPC, discloses combinations of prostaglandins, which may be F series prostaglandins, and clonidine derivatives.

An article in the Journal of Lipid Mediators, 6 (1993) 545-553 entitled "Intraocular pressure effects of selective prostanoid receptor agonists involve different receptor subtypes according to radioligand binding studies" discloses the use of prostaglandin analogues as tools for considering the classification of prostanoid receptors. The test method involves applying prostaglandin analogues to the eyes of a test animal for determining the competition of the agents at the various receptors.

### SUMMARY OF THE INVENTION

It has now been unexpectedly found that fluprostenol isopropyl ester shows significantly greater IOP reduction than the compounds of Stjernschantz et al., while having a similar or lower side effect profile. In particular, it appears that the addition of a trifluoromethyl group to the meta position on the phenoxy ring at the end of the omega chain provides a compound having excellent IOP reduction without the significant side effects found with other, closely related compounds.

In addition, it has also been unexpectedly found that fluprostenol isopropyl ester is useful in treating glaucoma and ocular hypertension. In particular, topical application of ophthalmic compositions comprising fluprostenol isopropyl ester result in significant IOP reduction.

### BRIEF DESCRIPTION OF THE DRAWING

Figure 1 is a graph showing the relative hyperemia scores (cumulative) of five tested compounds (see Table 2, below), one of which is a compound of the present invention.
Figure 2 is a graph showing the relative IOP-lowering effects of five tested compounds (see Table 2, below), one of which is a compound of the present invention. The dose for each of the tested compounds was 0.3 µg
Figure 3 is a graph similar to that of Figure 2, showing relative IOP-lowering effects of different concentrations of **A** (cloprostenol, isopropyl ester) and **E** (13,14-dihydro-17-phenyl-18,19,20-trinor PGF_{2α}, isopropyl ester).

### DETAILED DESCRIPTION OF THE INVENTION

The compound useful in the present invention is fluprostenl isopropyl esther.

Alkali metal salts and alkaline earth metal salts may be formed conventionally from the acid form. The acid may be converted to the ester by conventional condensation with an alcohol (isopropanol) or by reaction with an alkyl electrophile in the presence of base, according to known procedures. Since esterification reactions are well known, they are not further described here.

The compounds referred to herein include cloprostenol isopropyl ester (Table II, compound **A**), fluprostenol isopropyl ester (compound **B**), the 3-oxa form of cloprostenol isopropyl ester (Table 1, compound **5**), 13,14-dihydrofluprostenol isopropyl ester (compound **6**), cloprostenol-1-ol (compound **7**), and 13,14-dihydrocloprostenol-1-ol pivaloate (compound **8**) of which only fluprostenol isopropyl ester (compound **B**) is of the present invention.

Fluprostenol isopropyl ester is useful in lowering intraocular pressure and thus is useful in the treatment of glaucoma. The preferred route of administration is tapical. The dosage range for topical administration is generally between about 0.001 and about 1000 micrograms per eye (µg/eye) and is preferably between about 0.01 and about 100 µg/eye and most preferably between about 0.05 and 10 µg/eye. The compound of the present invention can be administered as solutions, suspensions, or emulsions (dispersions) in a suitable ophthalmic vehicle.

In forming compositions for topical administration, the compound of the present invention is generally formulated as between about 0.00003 to about 3 percent by weight (wt%) solutions in water at a pH between 4.5 to 8.0. The compound is preferably formulated as between about 0.0003 to about 0.3 wt% and, most preferably, between about 0.003 and about 0.03 wt%. While the precise regimen is left to the discretion of the clinician, it is recommended that the resulting solution be topically applied by placing one drop in each eye one or two times a day.

Other ingredients which may be desirable to use in the ophthalmic preparations of the present invention include preservatives, co-solvents and viscosity building agents.

### Antimicrobial Preservatives:

Ophthalmic products are typically packaged in multidose form, which generally require the addition of preservatives to prevent microbial contamination during use. Suitable preservatives include: benzalkonium chloride, thimerosal, chlorobutanol, methyl paraben, propyl paraben, phenylethyl alcohol, edetate disodium, sorbic acid, Onamer M®, or other agents known to those skilled in the art. Such preservatives are typically employed at a concentration between about 0.001% and about 1.0% by weight.

### Co-Solvents:

Prostaglandins, and particularly ester derivatives, typically have limited solubility in water and therefore may require a surfactant or other appropriate cosolvent in the composition. Such co-solvents include: Polysorbate 20, 60 and 80; Pluronic® F-68, F-84 and P-103; Tyloxapol®; Cremophort® EL, sodium dodecyl sulfate; glycerol; PEG 400; propylene glycol; cyclodextrins; or other agents known to those skilled in the art. Such co-solvents are typically employed at a concentration between about 0.01 % and about 2% by weight.

### Viscosity Agents:

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents known to those skilled in the art. Such agents are typically employed at a concentration between about 0.01% and about 2% by weight.

**Table 1**

| | **COMPOUND NAME** | **COMPOUND STRUCTURE** |
|---|---|---|
| **5** | 3-oxacloprostenol isopropyl ester | 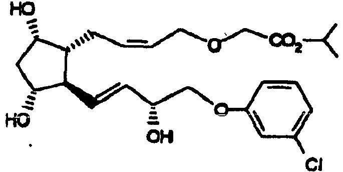 |
| **6** | 13,14-dihydrofluprostanol Isopropyl ester | 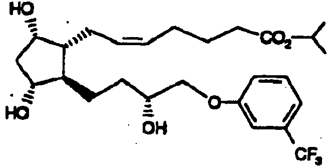 |
| **7** | cloprostenol-1-ol | 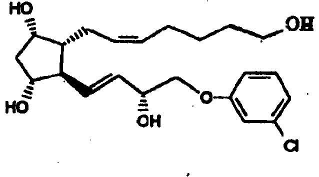 |
| **8** | 13,14-dihydrocloprostenol-1-ol pivaloate | 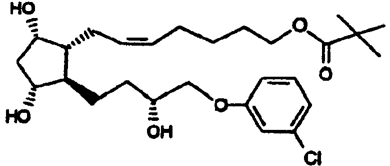 |

In the examples below, the following standard abbreviations are used: g = grams (mg = milligrams); mol = moles (mmol = millimoles); mol% = mole percent; mL = milliliters; mm Hg = millimeters of mercury; mp = melting point; bp = boiling point; h = hours; and min = minutes. In addition, "NMR" refers to nuclear magnetic resonance spectroscopy and "Cl MS" refers to chemical ionization mass spectrometry.

### EXAMPLE 1 (Reference): Synthesis of 3-Oxacloprostenol (5)

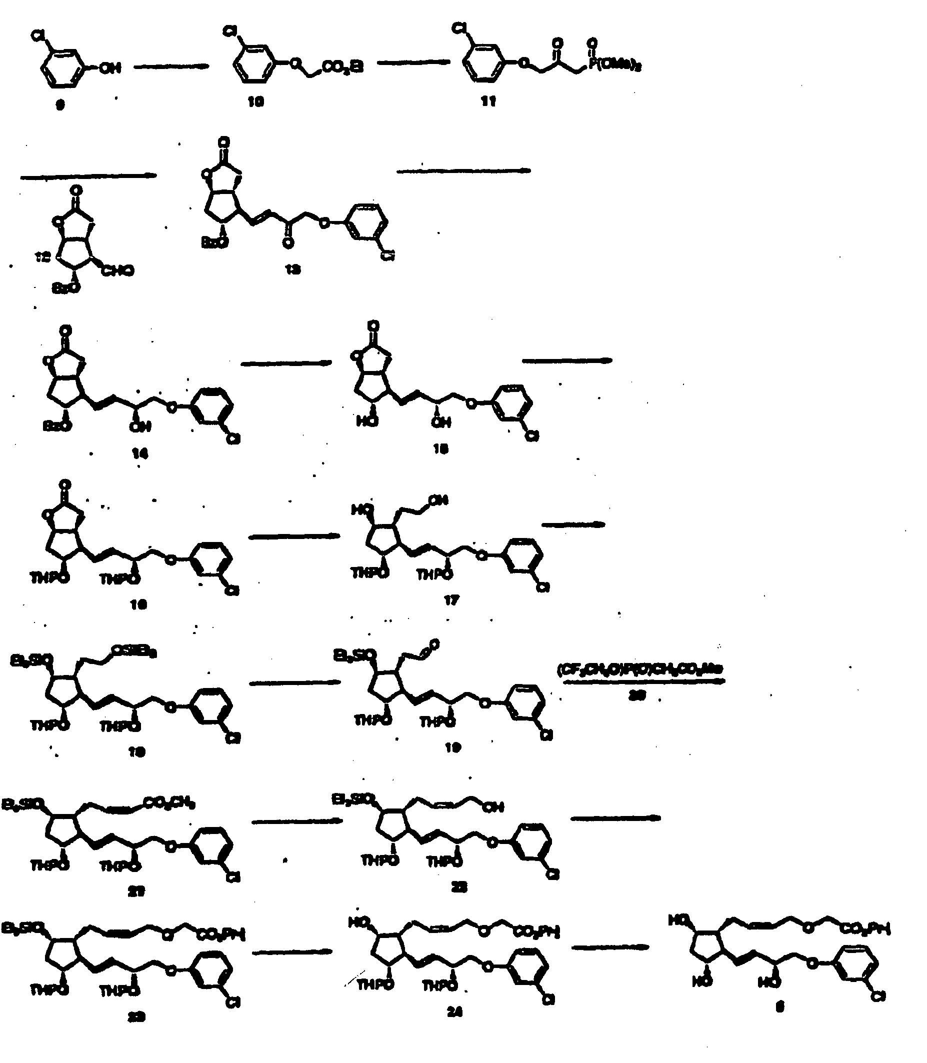

### A: Ethyl (3-chlorophenoxy)acetate (10)

Acetone (320 ml), 75 g (450 mmol) of ethyl bromoacetate, and 40.0 g (310 mmol) of 3-chlorophenol were mixed together, then 69.8 g (505 mmol) of potassium carbonate was added. The mixture was mechanically stirred and heated to reflux for 4 h, and after cooling to room temperature, was poured into 350 mL of ethyl acetate. To this was then cautiously added 400 mL of 1 *M* HCl, taking care to avoid excess foaming. The layers were separated and the aqueous layer was extracted with portions of ethyl acetate (3 X 200 mL). The combined organic layers were dried over MgSO₄, filtered, concentrated, and the resulting solid was recrystallized from hexane to afford 58 g (87%) of **10** as a white solid, m.p. = 39-40°C. ¹H NMR δ 7.20-7.08 (m, 1 H), 6.95-6.82 (m, 2 H), 6.75-6.70 (m, 1 H), 4.53 (s, 2 H), 4.21 (q, J = 7.2 Hz, 2 H), 1.23 (t, J = 7.2 Hz, 3 H).

### B: Dimethyl [3-(3-chlorophenoxy)-2-oxoprop-1-yl]phosphonate (11)

To 20.6 g (166 mmol, 238 mol%) of dimethyl methylphosphonate in 110 mL of THF at -78 °C was added dropwise 65 mL (162 mmol, 232 mol%) of a 2.5 M solution of *n*-BuLi in hexanes. After addition was complete, the mixture was stirred for an additional 1 h, after which 15.0 g (69.9 mmol) of aryloxyester **10** in 40 mL of THF was added dropwise. The reaction was stirred for 1 h and then quenched by the addition of 100 mL of saturated NH₄Cl. The mixture was poured into 200 mL of a 1/1 mixture of saturated NaCl/ethyl acetate, layers were separated, and the aqueous layer was further extracted with ethyl acetate (2 X 100 mL). Combined organic layers were dried over MgSO₄, filtered, and concentrated, to afford 20.5 g (100%) of **11** as a viscous oil. ¹H NMR δ 7.22 (t, J = 8.1 Hz, 1 H), 7.05-6.90 (m, 2 H), 6.85-6.78 (m, 1 H), 4.72 (s, 2 H), 3.84 (s, 3 H), 3.78 (s, 3 H), 3.27 (d, J = 22.8 Hz, 2 H).

### C: (3aR, 4R, 5R, 6aS)-5-(Benzoyloxy)-4-[(E)-4-(3-chlorophenoxy)-3-oxo-1-butenyl]-hexahydro-2H-cyclopenta[b]furan-2-one (13)

Phosphonate **11** (20.5 g, 70.0 mmol), 2.6 g (62 mmol) of LiCI, and 200 mL of THF were mixed together at 0 °C and 6.10 g (60.4 mmol) of NEt₃ was added. Aldehyde **12** (14.0 g, 51.1 mmol) dissolved in 50 mL of CH₂Cl₂ was then added dropwise. After 1 h, the reaction was poured into 200 mL of a 1/1 mixture of saturated NH₄Cl/ethyl acetate, the layers were separated, and the aqueous layer was extracted with ethyl acetate (2 X 100 mL). Combined organic layers were dried over MgSO₄, filtered, concentrated, and the residue was chromatographed on silica gel eluting with ethyl acetate/hexanes, 3/2, to afford 16.2 g (72%) of **13** as a white crystalline solid, m.p. = 101.0-102.0°C. ¹H NMR δ 8.0-7.9 (m, 2 H), 7.62-7.52 (m, 1 H), 7.50-7.38 (m, 2 H), 7.18 (t, J = 8.2 Hz, 1 H), 7.0-6.82 (m, 3 H), 6.75-6.70 (m, 1 H), 6.54 (d, J = 15.1 Hz, 1 H), 5.32 (q, J = 6.2 Hz, 1 H), 5.12-5.05 (m, 1 H), 4.66 (s, 2 H), 3.0-2.8 (m, 3 H), 2.7-2.2 (m, 3 H).

### D: (3aR, 4R, 5R, 6aS)-5-(Benzoyloxy)-4-[(E)-(3R)-4-(3-chlorophenoxy)-3-hydroxy-1-butenyl]-hexahydro-2H-cyclopenta[b]furan-2-one (14)

To a solution of 9.70 g (22.0 mmol) of enone **13** in 60 mL of THF at -23 °C was added dropwise a solution of 11.1 g (34.6 mmol of (-)-*B*-chlorodiisopino-campheylborane in 30 mL of THF. After 4 h, the reaction was quenched by the dropwise addition of 5 mL of methanol and then warmed to room temperature. After pouring into 200 mL of a 2/1 mixture of ethyl acetate/saturated NH₄Cl, the layers were separated, and the aqueous phase was extracted with ethyl acetate (2 X 100 mL). Combined organic layers were dried over MgSO₄, filtered, concentrated, and the residue was chromatographed on silica gel eluting with ethyl acetate/hexanes, 3/2, to afford 4.7 g (**48**%) of **14** as a white solid, m. p. 101.0-102.5 °C. ¹H NMR δ 8.05-7.95 (m, 2 H), 7.62-7.40 (m, 3 H), 7.18 (t, J = 8.0 Hz, 1 H), 7.0-6.92 (m, 1 H), 6.85 (t, J = 2.1 Hz, 1 H), 6.77-6.70 (m, 1 H), 5.85 (d of d, J = 6.2, 15.5 Hz, 1 H), 5.72 (d of d, J = 4.5, 15.5 Hz, 1 H), 5.30 (q, J = 5.8 Hz, 1 H), 5.12-5.04 (m, 1 H), 4.58-4.48 (m, 1 H), 3.92 (d of d, J = 3.5, 9.3 Hz, 1 H), 3.80 (d of d, J = 7.3, 9.4 Hz, 1 H), 2.9-2.2 (m, 8 H).

### E: (3aR, 4R, 5R, 6aS)-4-[(E)-(3R)-4-(3-Chlorophenoxy)-3-(tetrahydropyran-2-yloxy)-1-butenyl]-hexahydro-5-(tetrahydropyran-2-yloxy)-2H-cyclopenta[b]furan-2-one (16)

To a mixture of 5.1 g (11.5 mmol) of **14** in 200 mL of methanol was added 1.7 g (12 mmol) of K₂CO₃. After 1 h, the mixture was poured into 100 mL of 0.5 *M* HCl and extracted with ethyl acetate (3 X 100 mL). The combined organic layers were washed successively with water (2 X 100 mL) and saturated NaCI (2 X 100 mL). The organic layer was dried over MgSO₄, filtered, and concentrated to afford 4.85 g of crude diol **15**, which was used in the next step without further purification.

To a mixture of 4.85 g of crude **15** and 2.4 g (28 mmol) of 3,4-dihydro-2*H*-pyran in 75 mL of CH₂Cl₂ at 0 °C was added 370 mg (1.9 mmol) of *p*-toluenesulfonic acid monohydrate. After stirring for 45 min, the reaction was poured into 40 mL of saturated NaHCO₃, layers were separated, and the aqueous layer was extracted with CH₂Cl₂ (2 X 40 mL). The combined organic layers were dried over MgSO₄, filtered, and concentrated. The residue was chromatographed on silica gel eluting with 40% ethyl acetate in hexanes, to afford 6.0 g (100%) of **16** as an oil. ¹H NMR (CDCl₃) δ (characteristic peaks only) 7.25-7.14 (m, 1 H), 6.95-6.87 (m, 2 H), 6.83-6.72 (m, 1 H), 5.8-5.4 (m, 4 H), 5.1-4.8 (m, 2 H).

### F: (13E)-(9S, 11R, 15R)-11,15-Bis(tetrahydropyran-2-yloxy)-16-(3-chlorophenoxy)-2,3,4,5,6,17,18,19,20-nonanor-9-triethylsilyloxy-13-prostenol triethylsilyl ether (18)

To a suspension of 400 mg (10.5 mmol) of lithium aluminum hydride in 20 mL of THF at 0 °C was added dropwise a solution of 4.5 g (8.8 mmol) of lactone **16** in 20 mL of THF. After 1 h at 0 °C the mixture was cautiously poured into 100 mL of a 1/1 mixture of ice-cold saturated NH₄Cl/ethyl acetate. The layers were separated, and the aqueous layer was extracted with ethyl acetate (2 X 50 mL). The combined organic layers were dried over MgSO₄, filtered, and concentrated to afford 4.5 g (100%) of diol **17** which was used in the next step without further purification.

Triethylsilyl chloride (3.0 g, 20 mmol) was added to a mixture of 4.5 g (8.8 mmol) of crude **17**, 40 mL of DMF, 1.85 g (27.0 mmol) of imidazole, and 310 mg (2.5 mmol) of 4-(dimethylamino)pyridine. After 2 h, the reaction was poured into 100 mL of a 1/1 mixture of ethyl acetate/saturated NH₄Cl, layers were separated, and the aqueous layer was extracted with ethyl acetate (2 X 25 mL). The combined organic layers were washed with water (3 X 25 mL), dried over MgSO₄, and concentrated. The residue was chromatographed on silica gel eluting with 20% ethyl acetate in hexane to afford 5.2 g (80%) of **18**. ¹H NMR (CDCl₃) δ (characteristic peaks only) 7.22-7.12 (m, 1 H), 6.95-6.88 (m, 2 H), 6.83-6.71 (m, 1 H), 5.8-5.4 (m, 4 H), 5.1-4.8 (m, 2 H), 1.0-0.85 (m, 18 H), 0.7-0.5 (m, 12 H).

### G: (13E)-(9S, 11R, 15R)-11,15-Bis(tetrahydropyran-2-yloxy)-16-(3-chlorophenoxy)-2,3,4,5,6,17,18,19,20-nonanor-9-triethylsilyloxy-13-prostenal (19)

To a mixture of 1.6 g (12.6 mmol) of oxalyl chloride and 15 mL of CH₂Cl₂ at -78 °C was added dropwise a solution of 1.54 g (19.7 mmol) of DMSO in 2 mL of CH₂Cl₂. After 10 min, 4.6 g (6.2 mmol) of bissilane **18** in 8 mL of CH₂Cl₂ was added dropwise. After 95 min, 3.0 g (30 mmol) of NEt₃ was added. The mixture was then warmed to room temperature and poured into 70 mL of saturated NH₄Cl. The solution was extracted with of CH₂Cl₂ ( 3 X 70 mL) and the combined organic layers were dried over MgSO₄, filtered, and concentrated. The residue was chromatographed on silica gel eluting with 20% ethyl acetate in hexane to afford 2.06 g (53%) of **19** as well as 1.5 g (26%) recovered **18**. ¹H NMR (CDCl₃) δ (characteristic peaks only) 9.78 (t, J = 1.4 Hz, 1 H), 7.22-7.12 (m, 1 H), 6.95-6.88 (m, 2 H), 6.83-6.71 (m, 1 H), 5.8-5.4 (m, 4 H) 5.1-4.8 (m, 2 H), 1.0-0.85 (m, 18 H), 0.7-0.5 (m, 12 H).

### H: (5Z, 13E)-(9S, 11R, 15R)-11,15-Bis(tetrahydropyran-2-yloxy)-16-(3-chlorophenoxy)-2,3,4,17,18,19,20-heptanor-9-triethylsilyloxy-5,13-prostadienoic acid methyl ester (21)

To a solution of 1.35 g (4.24 mmol) of phosphonate **20** and 2.60 g (9.84 mmol) of 18-crown-6 in 20 mL of THF at -78 °C was added dropwise 6.9 mL (3.45 mmol) of a 0.5 *M* solution in toluene of potassium hexamethyldisilazane. After stirring for 15 min, a solution of 1.65 g (2.64 mmol) of aldehyde **19** in 20 mL of THF was added dropwise. One hour later, the mixture was poured into 100 mL of saturated NH₄Cl/ethyl acetate, 1/1, layers were separated, and the aqueous layer was extracted with ethyl acetate (3 X 30 mL). The combined organic layers were dried over MgSO₄, filtered, concentrated and the residue was chromatographed on silica gel eluting with 20% ethyl acetate in hexane to afford 1.135 g (63%) of **21**. ¹H NMR (CDCl₃) δ (characteristic peaks only) 7.22-7.11 (m, 1 H), 6.97-6.86 (m, 2 H), 6.85-6.75 (m, 1 H), 6.4-6.2 (m, 1 H), 5.8-5.32 (m, 3 H), 3.66 (s, 3 H).

### I: (5Z, 13E)-(9S, 11R, 15R)-11,15-Bis(tetrahydroxyran-2-yloxy)-16-(3-chlorophenoxy)-2,3,4,17,18,19,20-heptanor-9-triethylsilyloxy-5,13-prostadien-1-ol (22)

To a solution of 850 mg (1.25 mmol) of ester **21** in 10 mL of THF at 0 °C was added 2.4 mL (3.6 mmol) of a 1.5 *M* solution in toluene of diisobutylaluminum hydride. After 1 h, the mixture was poured into 20 mL of saturated NH₄Cl and was extracted with ethyl acetate (3 X 20 mL). Combined organic layers were dried over MgSO₄, filtered, and concentrated down to 800 mg (98%) of **22** as an oil. ¹H NMR (CDCl₃) δ (characteristic peaks only) 7.25-7.15 (m, 1 H), 6.97-6.90 (m, 2 H), 6.86-6.75 (m, 1 H), 5.81-5.41 (m, 4 H).

### J: (5Z, 13E)-(9S, 11R, 15R)-11,15-Bis(tetrahydropyran-2-yloxy)-16-(3-chlorophenoxy)-3-oxa-17,18,19,20-tetranor-9-triethylsilyloxy-5,13-prostadienoic acid isopropyl ester (23)

To a solution of 415 mg (6.37 mmol) of alcohol **22** in 4 mL of THF at -78 °C was added dropwise 0.35 mL (0.87 mol) of a 2.5 *M* solution in hexane of *n*-BuLi. After 15 min, this solution was transferred *via* syringe to a -78 °C solution of 195 mg (1.08 mmol) of isopropyl bromoacetate in 2 mL of THF. The mixture was kept at -78 °C for 40 min, warmed to room temperature overnight, and then poured into 20 mL of a 1/1 mixture of saturated NH₄Cl/ethyl acetate. Layers were separated, and the aqueous layer was extracted with ethyl acetate (2 X 10 mL). The combined organic layers were dried over MgSO₄, filtered, concentrated, and the residue was chromatographed on silica gel (20% ethyl acetate in hexane) to afford 242 mg (53%) of **23** as an oil. ¹H NMR (CDCl₃) δ (characteristic peaks only) 7.24-7.15 (m, 1 H), 6.97-6.90 (m, 2 H), 6.86-6.75 (m, 1 H), 5.81-5.41 (m, 4 H), 1.57 (d, J = 5.7 Hz, 6 H).

### K: (5Z, 13E)-(9S, 11R, 15R)-16-(3-Chlorophenoxy)-3-oxa-17,18,19,20-tetranor-9,11,15-trihydroxy-5,13-prostadienoic acid isopropyl ester (5)

To a solution of 230 mg (0.32 mmol) of silane **23** in 5 mL of THF at room temperature was added 0.33 mL (0.33 mmol) of a 1 *M* solution of Bu₄NF in THF. After 20 min, the reaction was poured into 4 mL of saturated NH₄Cl and was extracted with ethyl acetate (4 X 5 mL). The combined organic layers were dried over MgSO₄, filtered, concentrated, and the residue was chromatographed on silica gel (ethyl acetate/hexane, 1/1), to afford 126 mg (65%) of desilylated compound **24.**

To 120 mg of **24** in 5 mL of methanol was added 0.4 mL of 2 *M* HCl. After 1 h, the mixture was added to 3 mL of saturated NaHCO₃, and the resulting mixture was extracted with ethyl acetate (3 X 8 mL). Combined organic layers were dried over MgSO₄, filtered, concentrated. The resulting residue was then chromatographed on silica gel eluting with ethyl acetate to afford 54 mg (56%) of **5**. ¹³C NMR (CDCl₃) δ 169.92 (C), 159.26 (C), 135,13 (CH), 134.95 (CH), 134.81 (C), 124.93 (CH), 121.22 (CH), 115.06 (CH), 113.08 (CH), 77.75 (CH), 72.02 (CH), 71.94 (CH₂), 70.76 (CH₂), 68.77 (CH), 67.78 (CH₂), 66.50 (CH₂), 55.46 (CH), 49.93 (CH), 42.47 (CH₂), 25.85 (CH₂), 21.75 (CH₃), CI MS, *m*/*z* calcd. for C₂₄H₃₄O₇Cl₁ (MH⁺), 469.1993, found 469.1993.

### EXAMPLE 2 (Reference): Synthesis of 13,14-Dihydrofluprostenol Isopropyl Ester

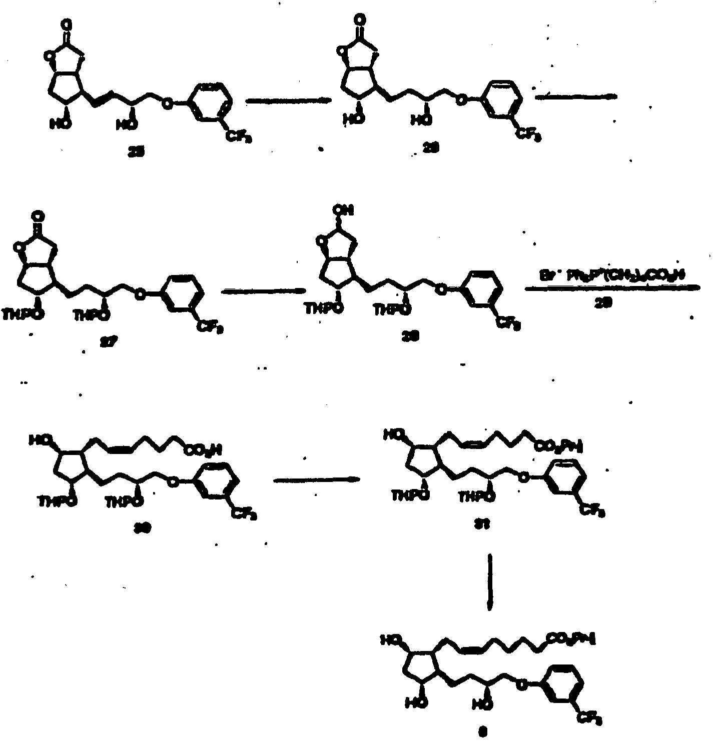

### A: (3aR, 4R, 5R, 6aS)-Hexahydro-5-hydroxy-4-[(3R)-4-(3-trifluoromethylphenoxy)-3-hydroxy-1-butyl] 2H cyclopenta[b]furan-2-one (26)

A mixture of 1.2 g (3.2 mmol) of diol **25** (for synthesis of diol **25**, see U.S. Patent 4,321,275) and 0.05 g of 10% (wt/wt) Pd/C in 20 mL of methanol was hydrogenated at 30 psi for 1.5 hours. After filtration through a short pad of Celite, concentration afforded 1.2 g of **26** as a colorless oil. ¹H NMR (CDCl₃) δ 7.44 (m, 2 H), 7.12 (m, 2 H), 4.95 (dt, 1 H), 4.15-3.80 (m, 4 H), 2.82 (dd, J = 10.8, 1 H), 2.55 (m, 2 H), 2.3 (m, 1 H), 2.1-1.3 (m, 6 H).

### B: (3aR, 4R, 5R, 6aS)-Hexahydro-5-(tetrahydropyran-2-yloxy)-4-[(3R)-4-(3-trifluoromethylphenoxy)-3- (tetrahydropyran-2-yloxy)-1-butyl]-2H-cyclopenta[b]furan-2-one (27)

A mixture of 1.2 g (3.2 mmol) of diol **26** and 0.05 g of *p*-toluenesulfonic acid monohydrate in 100 mL of CH₂Cl₂ at 0 °C was treated with dihydropyran (1.1 ml, 12 mmol) and the solution was stirred for 2 h at 0 °C. After pouring into saturated NaHCO₃, phases were separated and the organic layer was dried over MgSO₄, filtered, concentrated, and purified by chromatography on silica gel (1/1, hexanes/EtOAc) to afford 1.1 g of **27** as a clear, colorless oil. ¹H NMR (CDCl₃) δ 8.04 (dd, J = 7.0, 1.6, 1 H), 7.44 (m, 2 H), 7.12 (m, 1 H), 4.95 (dt, 1 H), 4.8 (m, 1 H), 4.7 (m, 2 H), 4.15-3.80 (m, 4 H), 3.5 (m, 2 H), 2.82 (dd, J = 10.8, 1 H), 2.55 (m, 2 H), 2.3 (m, 1 H), 2.1-1.3 (m, 6 H).

### C: (5Z)-(9S, 11R, 15 R)-11,15-Bis(tetrahydropyran-2-yloxy)-9-hydroxy-17,18,19,20-tetranor-16-(3-trifluoromethylphenoxy)-5-prostenoic acid isopropyl ester (31)

To a solution of 2.1 g (3.9 mmol) of **27** in 100 mL of THF at -78 °C was added 3.9 mL (5.8 mmol) of a 1.5 M solution of diisobutyaluminum hydride in toluene. The solution was stirred for 2 h, then quenched by the sequential addition of 0.4 mL of isopropanol at -78 °C followed by 0.4 mL of water at 23 °C. Volatiles were removed under reduced pressure and the aqueous solution was extracted with Et₂O/EtOAc (1/1). Organic extracts were dried over MgSO₄, filtered, and concentrated to furnish 1.9 g of lactol **28**.

To a 250 mL 3-necked round bottom flask equipped with a mechanical stirrer and a thermometer were added anhydrous DMSO (100 mL) and NaH (80% dispersion in mineral oil; 0.48 g, 16 mmol). The mixture was heated to 75 °C (internal) for 30 min, after which it was allowed to cool to room temperature for 1 h. Phosphonium bromide **29** (3.5 g, 8 mmol) was then added. After stirring for 30 minutes, 1.9 g (3.5 mmol) of lactol **28** in 50 mL of DMSO was added, and the resulting solution was heated to 50 °C for 2 h and then brought to room temperature for 16 h. The solution was then poured into 100 mL of water and approximately 2 mL of 50% NaOH added. The aqueous phase was extracted with ether (3 X 100 mL), then made acidic (pH = 5.5) by the addition of a 10% citric acid solution, and extracted with Et₂O:hexanes 2:1 (3 X 100 mL). The combined organic extracts were dried over MgSO₄, filtered, and concentrated to afford 1.9 g of **30** as a colorless oil.

To 1.9 g of carboxylic acid **30** dissolved in 10 mL acetone was added 0.95 g (6.0 mmol) of DBU and 1.0 g (6.1 mmol) of isopropyl iodide 1.0 g (6.1 mmol) at 23 °C. After 16 h, the solution was poured into 100 mL of water and extracted with 100 mL of EtOAc. The organic extract was dried over MgSO₄, filtered, concentrated, and purified by silica gel chromatography (3/2, hexanes/EtOAc) to afford 1.9 g of isopropyl ester **31** as a colorless oil. ¹H NMR (CDCl₃) δ 7.44 (t, 1 H), 7.12 (d, 1 H), 7.12 (dd, 2 H), 5.5-5.3 (m, 2 H), 4.99 (heptet, 1 H), 4.15-3.80 (m, 4 H), 2.82 (dd, J = 10.8, 1 H), 2.55 (m, 2 H), 2.3 (m, 1 H), 2.1-1.3 (m, 24 H), 1.23 (s, 3 H), 1.20 (s, 3 H).

### D: (5Z)-(9S, 11R, 15R)-17,18,19,20-Tetranor-16-(3-trifluoromethyl)-9,11,15-trihydroxy-5-prostenoic acid isopropyl ester (6)

Ester **31** (1.9 g, 2.8 mmol) was dissolved in 14 mL of a mixture of AcOH/THF/H₂O (4/2/1) and the solution was heated to 50 °C for 1 h, allowed to cool to 23 °C, poured into a saturated solution of NaHCO₃, and extracted with Et₂O (2 X 100 mL) and EtOAc (100 mL). The combined organic extracts were dried over MgSO₄, filtered, concentrated, and purified by silica gel chromatography (1/1, hexanes/EtOAc) to furnish 0.5 g of triol 6 as a clear, colorless oil. ¹H NMR (CDCl₃) δ 7.44 (t, J = 7.8, 1 H). 7.12 (dd, J = 7.8, 2.0, 1 H), 7.12 (ddd, J = 15.6, 7.2, 2.0, 2 H), 5.5-5.3 (m, 2 H), 4.99 (heptet, J = 6.3, 1 H), 4.15-3.80 (m, 4 H), 3.2 (d, 1 H), 2.95 (s, 1 H), 2.82 (dd, J = 10.8, 1 H), 2.75 (d, J = 5.9, 1 H), 2.55 (m, 2 H), 2.3 (m, 1 H), 2.1-1.3 (m, 24 H), 1.23 (s, 3 H), 1.20 (s, 3 H). CMR (CDCl₃) δ 173.5, 158.7, 132.1, 131.5, 130.0, 129.5, 129.2, 123.3, 120.8, 117.7, 117.6, 111.4, 111.4, 78.6, 74.4, 72.4, 69.9, 67.6, 52.6, 51.7, 42.5, 34.0, 31.5, 29.4, 26.8, 26.6, 24.9, 21.7.

### EXAMPLE 3 (Reference): Synthesis of Cloprostenol-1-ol (7)

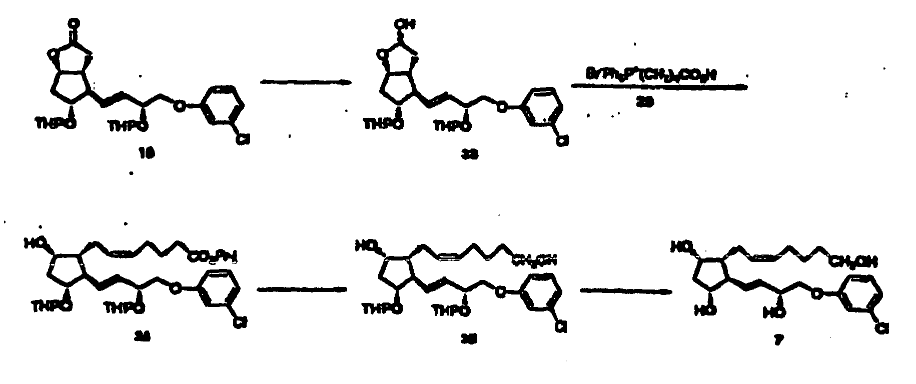

### A: (5Z, 13E)-(9S, 11R, 15R)-11,15-Bis(tetrahydropyran-2-yloxy)-16-(3-chlorophenoxy)-9-hydroxy-17,18,19,20-tetranor-5,13-prostadienoic acid isopropyl ester (34)

A 1.5 *M* solution of diisobutylaluminum hydride in toluene (10 mL, 15 mmol) was added dropwise to a solution of 5.8 g (11.4 mmol) of lactone **16** in **55** mL of THF at -78 °C. After 1 h, 10 mL of methanol was added dropwise, and the mixture was stirred for 10 min at -78 °C before being warmed to room temperature. The mixture was then poured into 100 mL of a 1/1 solution of saturated aqueous potassium sodium tartrate/ethyl acetate and stirred. After separating layers, the aqueous phase was extracted with ethyl acetate (2 X 40 mL). Combined organic layers were dried over MgSO₄, filtered, concentrated, and purified by silica gel chromatography (3/2, ethyl acetate/hexane), to afford 4.4 g (76%) of lactol **33**, which was used immediately in the next step.

A 1 *M* solution of potassium t-butoxide in THF (50.0 ml) was added dropwise to 12.1 g (27.3 mmol) of phosphonium salt **29** in 100 mL of THF at 0 °C. After 30 min, a solution of 4.4 g (8.6 mmol) of lactol **33** in 20 mL of THF was added dropwise, and the mixture was stirred at room temperature overnight. The solution was then poured into 150 mL of a 1/1 mixture of ethyl acetate/saturated NH₄Cl. Layers were separated and the aqueous layer was extracted with ethyl acetate (2 X 100 mL). Combined organic layers were dried over MgSO₄, filtered, concentrated, and the residue was redissolved in 80 mL of acetone. To this was added 6.5 g (45 mmol) of DBU followed by 7.3 g (43 mmol) of isopropyl iodide. After stirring overnight, the reaction was poured into 100 mL of a 1/1 mixture of ethyl acetate/saturated NH₄Cl. Layers were then separated and the aqueous phase was further extracted with ethyl acetate (2 X 100 mL). The combined organic layers were dried over MgSO₄, filtered, concentrated, and purified by silica gel chromatography (40% ethyl acetate in hexane) to afford 2.92 g (53% from lactone **16**) of ester **34**.

### B: (5Z, 13E)-(9S, 11R, 15R)-16-(3-Chlorophenoxy)-17,18,19,20-tetranor-9,11,15-trihydroxy-5,13-prostadienol (7)

A solution of 500 mg (0.79 mmol) of **34** in 10 mL of THF was added dropwise to 61 mg (1.60 mmol) of lithium aluminum hydride in 20 mL of THF at 0 °C. After 40 min, the reaction was poured into 15 mL of saturated NH₄Cl, and the mixture was then extracted with ethyl acetate ( 3 X 40 mL). Combined organic layers were dried over MgSO₄, filtered, and concentrated to afford 500 mg of crude **35**.

To a solution of 500 mg of **35** in 20 mL of methanol was added 0.5 mL of 2 *M* HCl. After 1 h, the reaction was quenched with 20 mL of saturated NaHCO₃ and the mixture was extracted with ethyl acetate (4 X 30 mL). The combined organic layers were dried over MgSO₄, filtered, and concentrated. Silica gel chromatography (EtOAc) provided 101 mg (31% from **34**) of **7**. ¹³C NMR (CDCl₃) δ 159.27 (C), 135.44 (CH), 134.82 (C), 130.64 (CH), 130.26 (CH), 128.23 (CH), 121.25 (CH), 115.07 (CH), 113.08 (CH), 77.35 (CH), 72.35 (CH), 71.90 (CH₂), 70.89 (CH), 62.22 (CH₂), 55.40 (CH), 49.87 (CH), 42.79 (CH₂), 31.83 (CH₂), 26.77 (CH₂), 25.60 (CH₂), 25.33 (CH₂). CI MS *m*/*z* calcd for C₂₂H₃₂O₅Cl₁ (MH⁺) 411.1938, found 411.1938.

### EXAMPLE 4 (Reference): Synthesis of 13,14-Dihydrocloprostenol-1-ol Pivaloate (8)

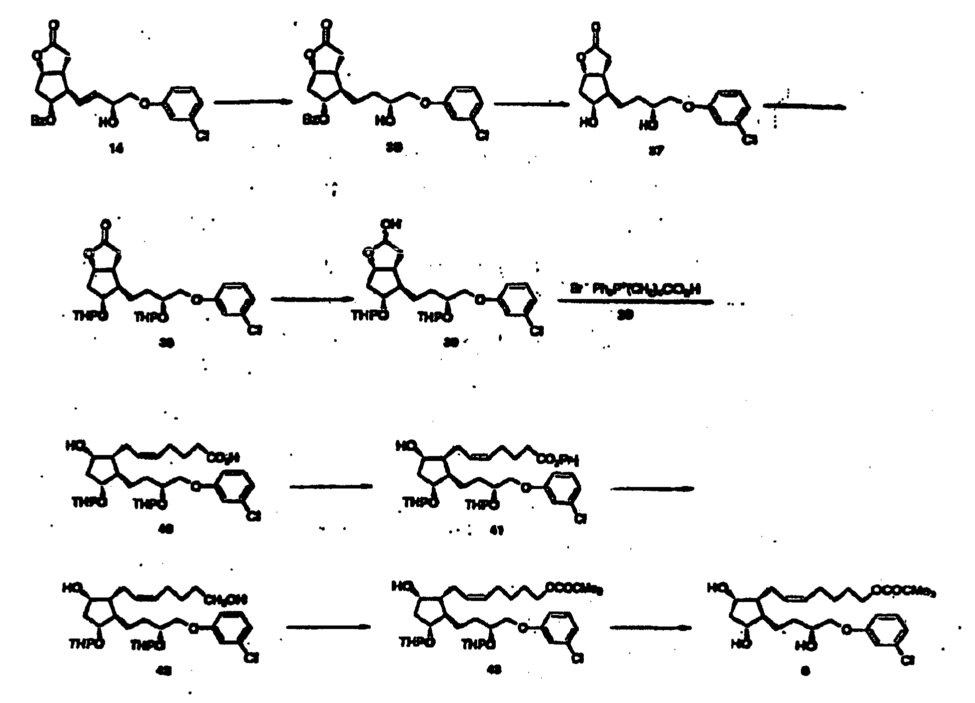

### A: (3aR, 4R, 5R, 6aS)-4-[(3R)-4-(3-Chlorophenoxy)-3-hydroxybutyl]-hexahydro-5-hydroxy-2H-cyclopenta[b]furan-2-one (37):

A mixture of 2.4 g (5.4 mmol) of **14** and 250 mg of 10% (wt/wt) Pd/C in 35 mL of ethyl acetate was hydrogenated at 40 psi for 1 h. After filtration through a short pad of Celite, the filtrate was evaporated down to 2.3 g (100%) of hydrogenated product **36**.

The crude benzoate **36** was dissolved in 25 mL of methanol, and 610 mg (4.4 mmol) of K₂CO₃ was added. After 3.5 h, the mixture was poured into 100 mL of water/ethyl acetate (1/1). Layers were separated, and the aqueous phase was further extracted with ethyl acetate (2 X 50 mL). The combined organic layers were dried over MgSO₄, filtered and concentrated. Silica gel chromatography (EtoAc) provided 1.50 g (82%) of **37** as a white solid, m.p. = 102.0-103.5 °C. ¹H NMR δ 7.22 (t, J = 8.2 Hz, 1 H), 7.0-6.94 (m, 1 H), 6.91-6.88 (t, J = 2.1 Hz, 1 H), 6.83-6.77 (m, 1 H), 4.97 (dt, J = 3.0, 8.3 Hz, 1 H), 4.12-3.91 (m, 3 H), 3.82 (dd, J = 7.4, 9.0 Hz, 1 H), 2.85 (dd, J = 8.0, 16.5 Hz, 1 H), 2.6-1.4 (m, 11 H).

### B: (3aR, 4R, 5R, 6aS)-4-[(3R)-4-(3-Chlorophenoxy)-3-(tetrahydropyran-2-yloxy)butyl]-hexahydro-5-(tetrahydropyran-2-yloxy)-2H-cyclopenta[b]furan-2-one (38)

Diol **37** (3.4 g, 10 mmol) and 2.2 g (26 mmol) of 3,4-dihydro-2*H*-pyran were dissolved in 80 mL of CH₂Cl₂, and 240 mg (1.3 mmol) of *p*-toluenesulfonic acid monohydrate was added at 0 °C. After 1 h, the reaction was poured into 50 mL of saturated NaHCO₃ and the mixture was extracted with CH₂Cl₂ (3 X 40 mL). The combined organic layers were dried over MgSO₄, filtered, concentrated, and the residue was chromatographed on silica gel (hexane/ethyl acetate, 1/1) to afford 4.5 g (87%) of bis-THP ether **38**.

### C: (5Z)-(9S, 11R, 15R)-11,15-Bis(tetrahydropyran-2-yloxy)-16-(3-chlorophenoxy)-9-hydroxy-17,18,19,20-tetranor-5-prostenoic acid isopropyl ester (41)

A 1.5 *M* solution of diisobutylaluminum hydride in toluene (1.8 mL, 2.7 mmol) was added to the solution 1.05 g (2.06 mmol) of **38** in 10 mL of THF at -78 °C. After 1 h, 4 mL of methanol was added and the mixture was warmed to 25 °C, then poured into 40 mL of ethyl acetate/saturated aqueous potassium sodium tartrate (1/1). Layers were separated and the aqueous phase was further extracted with ethyl acetate (3 X 30 mL). The combined organic layers were then dried over MgSO₄, filtered, concentrated, and the residue was chromatographed on silica gel (ethyl acetate) to afford 740 mg (70%) of lactol **39**.

A 1.5 *M* solution of potassium t-butoxide in THF (8.6 mL, 8.6 mmol) was added dropwise to a mixture of 15 mL of THF and 1.92 g (4.33 mmol) of phosphonium salt **29** at 0 °C. After stirring 1 h, a solution of 740 mg (1.45 mmol) of lactol **39** in 5 mL of THF was added dropwise, and the reaction was allowed to warm to 25 °C overnight. The mixture was then poured into 100 mL of ethyl acetate/saturated NH₄Cl (1/1). Layers were separated, and the aqueous phase was further extracted with ethyl acetate (2 X 70 mL). Combined organic layers were dried over MgSO₄, filtered, and concentrated to afford 1.6 g of crude acid **40**.

Crude acid **40** (1.6 g) was dissolved in 11 mL of acetone and cooled to 0 °C, then 850 mg (5.6 mmol) of DBU was added dropwise to the solution. The resulting mixture was stirred for 15 min at 0 °C and 30 min at 25 °C, after which 850 mg (5.0 mmol) of isopropyl iodide was added. The reaction was stirred overnight, poured into 100 mL of ethyl acetate/saturated NH₄Cl (1/1). Layers were separated, and the aqueous phase was further extracted with ethyl acetate (2 X 50 mL). Combined organic layers were dried over MgSO₄, filtered and concentrated. The resulting residue was purified by silica gel chromatography (ethyl acetate/hexanes, 3/2) to afford 560 mg (61% from lactol **39**) of isopropyl ester **41**.

### D: (5Z)-(9S, 11R, 15R)-16-(3-Chlorophenoxy)-17,18,19,20-tetranor-9,11,15-trihydroxy-5-prostenyl pivaloate (8)

A solution of 400 mg (0.63 mmol) of **41** in 5 mL of THF was added dropwise to a suspension of 35 mg (0.92 mmol) of lithium aluminum hydride in **5** mL of THF at 0 °C. After 2 h, the reaction was poured into 50 mL of a 1/1 mixture of ethyl acetate/saturated NaHCO₃. The layers were then separated, and the aqueous phase was extracted with ethyl acetate (2 X 2 mL). Combined organic layers were dried over MgSO₄, filtered, and concentrated. The resulting residue purified by silica gel chromatography (ethyl acetate) to afford 350 mg (95%) of diol **42**.

Pivaloyl chloride (90 mg, 0.75 mmol) was added to a mixture of 350 mg (0.60 mmol) of **42**, 60 mg (0.76 mmol) of pyridine, 22 mg (0.18 mmol) of 4-(dimethylamino)pyridine, and 7 mL of CH₂Cl₂. After 1.5 h the mixture was poured into 30 mL of saturated NH₄Cl/ethyl acetate (1/1). Layers were then separated and the aqueous phase was extracted with ethyl acetate (2 X 20 mL). The combined organic layers were dried over MgSO₄, filtered, concentrated, and purified by silica gel chromatography (ethyl acetate/hexane, 3/2) to afford 370 mg (93%) of pivaloate **43.**

Water (10 drops) and concentrated HCl (3 drops) were added to a solution of 370 mg (0.56 mmol) of **43** in 5 mL of methanol. After stirring overnight, the reaction was quenched by the addition of 20 mL of saturated NaHCO₃, and the mixture was extracted with ethyl acetate (3 X 20 mL). The combined organic layers were dried over MgSO₄, filtered, and concentrated. The residue was chromatographed on silica gel (ethyl acetate/hexane, 3/2), to afford 165 mg (59%) of triol **8**. ¹³C NMR (CDCl₃) δ 178.77 (C), 159.27 (C), 134.80 (C), 130.20 (CH), 128.62 (CH), 121.19 (CH), 114.97 (CH), 112.97 (CH), 78.50 (CH), 74.46 (CH), 72.31 (CH₂), 69.86 (CH), 64.16 (CH₂), 52.53 (CH), 51.67 (CH), 42.50 (CH₂), 31.51 (CH₂), 29.40 (CH₂), 28.10 (CH₂), 27.12 (CH₃), 26.77 (CH₂), 26.65 (CH₂), 25.77 (CH₂). Cl MS, *m*/*z* calcd for C₂₇H₄₁O₆Cl₁ (MH⁺), 497.2670, found 497.2656

The studies detailed in the following Examples 5-9 compared the IOP lowering activity and side effects of five compounds: A) Cloprostenol, isopropyl ester; B) Fluprostenol, isopropyl ester; C) 16-Phenoxy-17,18,19,20-tetranor PGF_{2α}, isopropyl ester; D) 17-Phenyl-18,19,20-trinor PGF_{2α}, isopropyl ester; and E) 13,14-Dihydro-17-phenyl-18,19,20-trinor PGF_{2α}, isopropyl ester (latanoprost). The structures of these compounds are shown in the following Table 2.

**Table 2**

| | **COMPOUND NAME** | **COMPOUND STRUCTURE** |
|---|---|---|
| **A** | Cloprostenol, isopropyl ester | 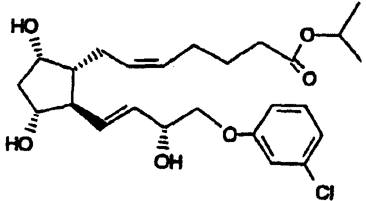 |
| **B** | Fluprostenol, isopropyl ester | 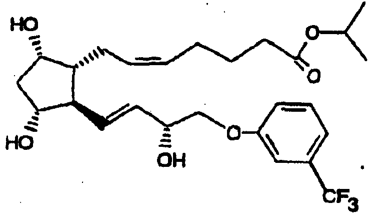 |
| **C** | 16-Phenoxy-17,18,19,20-tetranor PGF_{2α}, isopropyl ester | 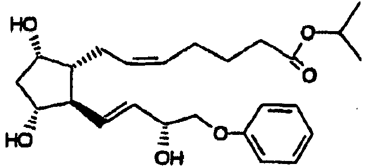 |
| **D** | 17-Phenyl-18,19,20-trinor PGF_{2α}, isopropyl ester | 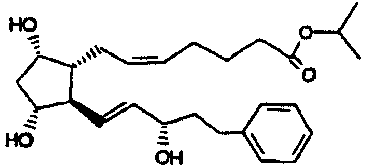 |
| **E** | 13,14-Dihydro-17-phenyl-18,19,20-trinor PGF_{2α}, isopropyl ester | 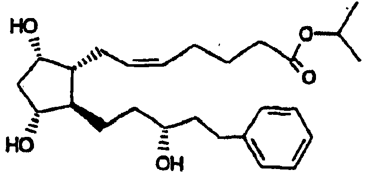 |

As is apparent in Table 2, the five compounds differ only slightly in structure; however, as Examples 5 and 6 will show, such seemingly slight structural differences produce greatly different IOP-lowering effects and levels of hyperemia.

### EXAMPLE 5

Compounds **A-E** (Table 2, above) were tested for hyperemia in the guinea pig. The objective of the guinea pig conjunctival hyperemia model is to provide a primary screening indication of the potential of a prostaglandin for inducing conjunctival hyperemia in humans.

Guinea pigs were maintained in their cages during the study, and removed only for scoring and dosing. Eyes were evaluated using a magnifying lens with fluorescent illumination and scores for conjunctival hyperemia were recorded for upper bulbar conjunctiva according to the following criteria:
- *0 =*: *Normal appearance of vessels at limbus and on superior rectus muscle*
- *+1 =*: *Enlargement of vessels normally visible at limbus and on superior rectus muscle*
- *+2 =*: *Branch of vessels at limbus, new vessels visible*
- *+3 =*: *New vessels visible in open bulbar conjunctival areas*
- *+4 =*: *Diffuse redness in open bulbar conjunctival areas*
Scores of 0 or 1 indicated no hyperemia, and scores of 2-4 indicated hyperemia (a score of 4 indicating the most hyperemia). Only integer scores were permitted in order to minimize subjectivity.

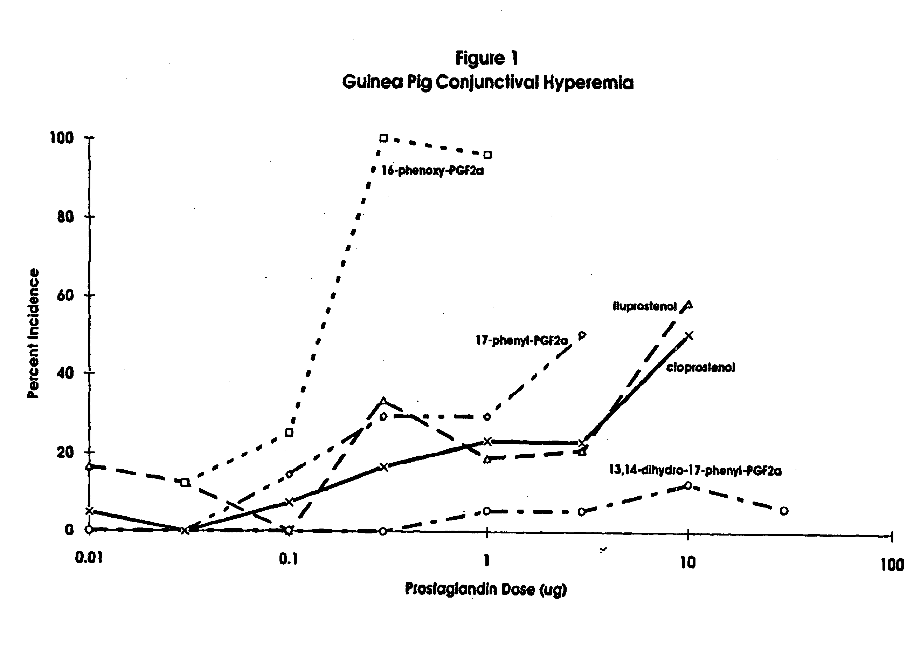

Baseline observations were made prior to unilateral dosing with a 10 µL aliquot of either the prostaglandin test formulation or the control formulation, followed by observations at 1, 2, 3 and 4 hours after dosing. Groups typically contained four animals, but ranged up to eight animals per group. The results of the study are presented in Table 3, below, as percent frequency of each score, and in Figure 1 as percent incidence of hyperemia, defined as the percent of scores of +2 or +3 relative to the total number of observations for each dose.

**Table 3: Guinea Pig Conjunctival Hyperemia****

| **Compound** (isopropyl ester) | **Prostaglandin Dose** | | | | | | | | | | | | | | | | | | | |
|---|---|---|---|---|---|---|---|---|---|---|---|---|---|---|---|---|---|---|---|---|
| | **0.03 µg** | | | | | **0.1 µg** | | | | | **0.3 µg** | | | | | **1.0 µg** | | | | |
| | Score | | | | N* | Score | | | | N* | Score | | | | N* | Score | | | | N* |
| | 0 | 1 | 2 | 3 | | 0 | 1 | 2 | 3 | | 0 | 1 | 2 | 3 | | 0 | 1 | 2 | 3 | |
| **A** (Cloprostanol) | 40 | 60 | 0 | 0 | 5 | 60 | 33 | 7 | 0 | 23 | 23 | 61 | 13 | 3 | 21 | 18 | 59 | 19 | 4 | 23 |
| **B** (Fluprostanol) | 17 | 70 | 13 | 0 | 6 | 12 | 88 | 0 | 0 | 6 | 17 | 50 | 29 | 4 | 6 | 21 | 60 | 13 | 6 | 12 |
| **C** (16-Phenoxy-17,18,19,20-tetranor PGF_{2α}) | 33 | 54 | 13 | 0 | 6 | 4 | 71 | 25 | 0 | 6 | 0 | 0 | 62 | 38 | 6 | 0 | 4 | 33 | 63 | 6 |
| **D** (17-Phenyl-16,19·20-trinor PGF_{2α}) | 46 | 54 | 0 | 0 | 6 | 23 | 62 | 13 | 2 | 12 | 10 | 61 | 27 | 2 | 12 | 15 | 56 | 17 | 12 | 12 |
| **E** (13,14-Dihydro-17-phenyl-18,19,20-trinor PGF_{2α}) | 80 | 20 | 0 | 0 | 5 | 75 | 25 | 0 | 0 | 5 | 40 | 60 | 0 | 0 | 5 | 39 | 56 | 6 | 0 | 9 |

| | | | | | | | | | | | | | | | | | | | | |
|---|---|---|---|---|---|---|---|---|---|---|---|---|---|---|---|---|---|---|---|---|
| *Number of animals tested **Numbers Indicate percent Incidence for that score | | | | | | | | | | | | | | | | | | | | |

### Discussion:

Compound **C** (16-phenoxy-17,18,19,20-tetranor PGF_{2α}, isopropyl ester) produces significant hyperemia at low doses, and at 0.3 and 1.0 µg doses, all eyes received one or more scores of +3. Compound **D** (17-phenyl-18,19,20-trinor PGF_{2α}, isopropyl ester) produces less hyperemia than compound **C**, but significantly more than compound **E** (13,14-dihydro-17-phenyl-18,19,20-trinor PGF_{2α}, isopropyl ester), which produces only mild hyperemia. The hyperemia produced by compound **A** (cloprostenol, isopropyl ester) and compound **B** (fluprostenol, isopropyl ester) appear to be intermediate between that of compound **D** and compound **E**.

### EXAMPLE 6

In the study presented below, compounds **A-E** (Table 2, above) were tested for IOP-lowering effect in cynomolgus monkey eyes.

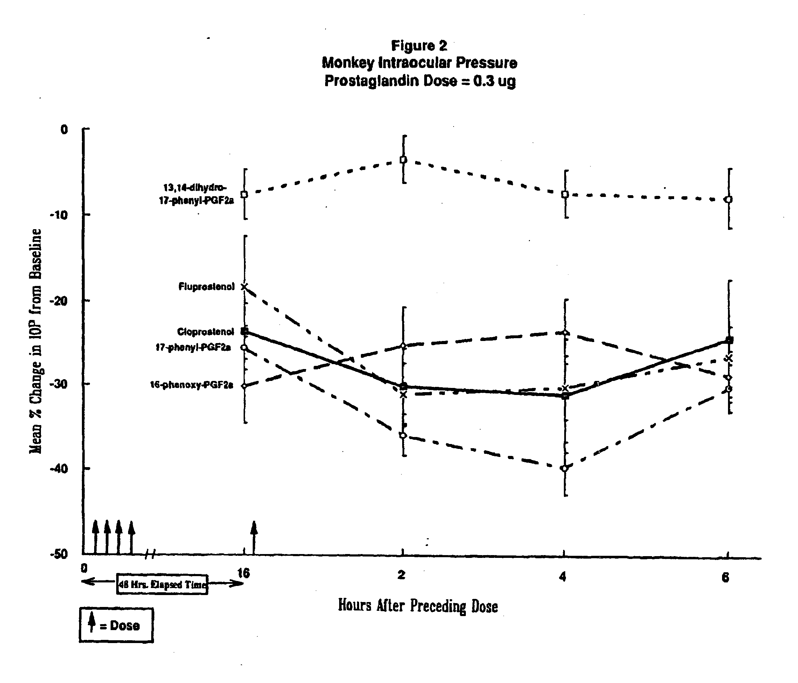

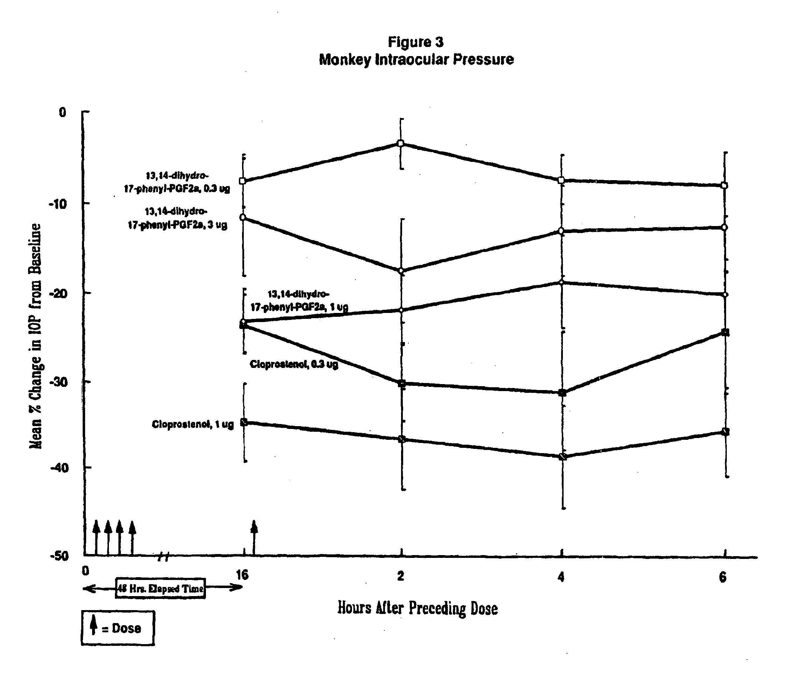

The right eyes of the cynomolgus monkeys used in this study were previously given laser trabeculoplasty to induce ocular hypertension in the lasered eye. Animals had been trained to sit in restraint chairs and conditioned to accept experimental procedures without chemical restraint. IOP was determined with a pneumatonometer after light corneal anesthesia with dilute proparacaine. The test protocol included a five-dose treatment regimen because of the typical delayed response to prostaglandins. The designated test formulations were administered to the lasered right eyes, and the normal left eyes remained untreated, although IOP measurements were taken. Baseline IOP values were determined prior to treatment with the test formulation, and then IOP was determined from 1 to 7 hours after the first dose, 16 hours after the fourth dose, and 1 to 4 hours after the fifth dose. Results are presented in Tables 4 and 5, below, and in Figures 2 and 3, as the mean percent reduction of IOP from baseline ± SEM. Prostaglandin doses are micrograms of compound contained in each treatment with 10 µL of the test formulation. In Table 4, the same amount (0.3 µg) of each of compounds **A-E** were compared for IOP reduction. In Table 5, various amounts of compound **A** (0.3 and 1.0 µg) were compared against various amounts of compound **E** (0.3, 1.0 and 3.0 µg) in order to determine the dose responses of the two different compounds.

**Table 4: Percent IOP Reduction in Lasered Cynomolgus Monkeys**

| Compound (isopropyl ester) | Baseline IOP (mm Hg) | Percent IOP Reduction (Hours after Last Dose/Dose#) | | | |
|---|---|---|---|---|---|
| | | 16/4 | 2/5 | 4/5 | 6/5 |
| **A** (Cloprostenol) | 36.9 | 23.6 ± 3.3 | 30.2 ± 4.5 | 31.2 ±6.8 | 24.4 ± 6.9 |
| **B** (Fluprostenol) | 41.6 | 18.4 ± 5.9 | 31.2 ± 3.7 | 30.3 ± 3.8 | 26.6 ± 3.6 |
| **C** (16-Phenoxy-17,18,19,20-tetranor PGF_{2α}) | 38.2 | 30.2 ± 4.4 | 25.3 ± 4.5 | 23.6 ± 3.8 | 28.9 ± 3.0 |
| **D** (17-Phenyl-18, 19,20-trinor PGF_{2α}) | 40.8 | 25.6 ± 2.6 | 36.0 ± 2.4 | 39.8 ± 3.1 | 30.3 ± 2.8 |
| **E** (13,14-Dihydro-17-phenyl-18,19, 20-minor PGF_{2α}) | 39.7 | 7.6 ± 2.9 | 3.6 ± 2.7 | 7.5 ± 2.7 | 8.0 ± 3.4 |

**Table 5: Comparison of Percent IOP Reduction**

| **Compound** | Dose (µg) | Baseline IOP (mm Hg) | Percent IOP Redudion (Hours after Last Dose/Dose#) | | | |
|---|---|---|---|---|---|---|
| | | | 16/4 | 2/5 | 4/5 | 6/5 |
| **A*** | 0.3 | 36.9 | 23.6 ± 3.3 | 30.2 ± 4.5 | 31.2 ± 6.8 | 24.4 ± 6.9 |
| **A** | 1 | 39.6 | 34.8 ± 45 | 36.7 ± 5.8 | 38.7 ± 5.9 | 35.8 ± 5.1 |
| **E** | 0.3 | 39.7 | 7.6 ± 2.9 | 3.6 ± 2.7 | 7.5 ± 2.7 | 8.0 ± 3.4 |
| **E**** | 1 | 38.9 | 23.2 ± 3.8 | 22.0 ± 4.0 | 18.8 ± 5.2 | 20.2 ± 4.0 |
| **E** | 3 | 30.1 | 11.6 ± 6.5 | 17.8 ± 5.8 | 13.1 ± 5.0 | 12.7 ± 5.0 |

| | | | | | | |
|---|---|---|---|---|---|---|
| *Cloprostenol, isopropyl ester **13,14-Dihydro-17-phenyl-18,19.20-trinor PGF_{2α}, Isopropyl ester | | | | | | |

### Discussion:

Table 4 shows that compounds **A, B, C,** and **D** produce similar degrees of IOP reduction with 0.3 µg doses; however, compound **E** is essentially inactive at this dose.

In Table 5, it is apparent that the IOP reduction with 1 µg of compound **A** is greater than that produced by 0.3 µg of compound **A**, and the response to either of these doses of compound **A** is greater than the maximum reduction produced by either dose of compound **E**. These observations indicate that compound **A** (cloprostenol, isopropyl ester) is both more potent and produces a greater maximum response for IOP reduction than compound **E** (13,14-dihydro-17-phenyl-18,19,20-trinor PGF_{2α}).

### EXAMPLE 7

PGF_{2α} analogues are known to contract the iris sphincter of cats and this assay is a generally accepted reference for activity. For this reason, the pupil diameter of cats may be used to define the activity of PGF_{2α} analogues and, as demonstrated by Stjernschantz and Resul (Drugs Future, 17:691-704 (1992)), predict the IOP-lowering potency.

Compounds of the present invention were therefore screened for pupillary constriction in the cat. Data for compounds **6**, **7**, and **8** are presented in Table **6**, below. The response is quantitated as Area ₁₋₅ values (area under the pupil diameter versus time curve from 1-5 hours), and the equivalent response dose (ED₅) is estimated from its dose response relationship.

**Table 6:**

| **Cat Pupil Diameter Response** | |
|---|---|
| **Compound** | **ED₅ (µg)** |
| PGF_{2α} Isopropyl Ester | 0.02 |
| Cloprostenol Isopropyl Ester | 0.01 |
| **6** | 0.2 |
| **7** | 0.02 |
| **8** | 0.06 |

### Discussion:

The two standard compounds, PGF_{2α} isopropyl ester and cloprostenol isopropyl ester, produced marked change in cat pupillary diameter, displaying ED₅ values of 0.02 and 0.01 µg, respectively. Compound **7** (cloprostenol-1-ol) and compound **8** (13,14-dihydrocloprostenol-1-ol pivaloate), displayed nearly equivalent potency. 13,14-Dihydrofluprostenol isopropyl ester (compound **6**) was approximately one order of magnitude less potent, with an ED₅ of 0.2 µg.

### EXAMPLE 8

In the study presented below, compound **6** (Table 1, above) was tested for IOP-lowering effect in cynomolgus monkey eyes.

The right eyes of the cynomolgus monkeys used in this study were previously given laser trabeculoplasty to induce ocular hypertension in the lasered eye. Animals had been trained to sit in restraint chairs and conditioned to accept experimental procedures without chemical restraint. IOP was determined with a pneumatonometer after light corneal anesthesia with dilute proparacaine. The test protocol included a five-dose treatment regimen because of the typical delayed response to prostaglandins. The designated test formulations were administered to the lasered right eyes, and the normal left eyes remained untreated, although IOP measurements were taken. Baseline IOP values were determined prior to treatment with the test formulation, and then IOP was determined from 1 to 7 hours after the first dose, 16 hours after the fourth dose, and 1 to 4 hours after the fifth dose.

The equivalent response dose (ED₂₀) is estimated from the dose response relationship to be the dose producing a 20% peak reduction in IOP.

**Table 7:**

| **Monkey lop Response** | |
|---|---|
| **Compound** | **ED₂₀ (µg)** |
| PGF_{2α} Isopropyl Ester | 0.4 |
| **6** | 0.3 |

### Discussion:

As can be seen in Table 7, above, compound **6**, the 13,14-dihydro analogue of fluprostenol was quite potent in the monkey IOP model, producing a 20% reduction at 0.3 µg. This was even more potent than the standard compound, PGF_{2α} isopropyl ester.

### EXAMPLE 9

The following Formulations 1-8 are pharmaceutical compositions for topical use in lowering of intraocular pressure although Formulations 1-3 and 5-8 are included for reference only. Each of Formulations 1 through 8 may be formulated in accordance with procedures known to those skilled in the art.

| FORMULATION 1 | |
|---|---|
| **Ingredient** | **Amount (wt%)** |
| Cloprostenol isopropyl ester (Table 2, Compound **A**) | 0.002 |
| Dextran 70 | 0.1 |
| Hydroxypropyl methylcellulose | 0.3 |
| Sodium Chloride | 0.77 |
| Potassium chloride | 0.12 |
| Disodium EDTA (Edetate disodium) | 0.05 |
| Benzalkonium chloride | 0.01 |
| HCl and/or NaOH | pH 7.2 - 7.5 |
| Purified water | q.s. to 100% |

| FORMULATION 2 | |
|---|---|
| **Ingredient** | **Amount (wt%)** |
| Cloprostenol, *t*-butyl ester | 0.01 |
| Monobasic sodium phosphate | 0.05 |
| Dibasic sodium phosphate (anhydrous) | 0.15 |
| Sodium chloride | 0.75 |
| Disodium EDTA (Edetate disodium) | 0.01 |
| Benzalkonium chloride | 0.02 |
| Polysorbate 80 | 0.15 |
| HCl and/or NaOH | pH 7.3 - 7.4 |
| Purified water | q.s. to 100% |

| FORMULATION 3 | |
|---|---|
| **Ingredient** | **Amount (wt%)** |
| Cloprostenol, methyl ester | 0.001 |
| Dextran 70 | 0.1 |
| Hydroxypropyl methylcellulose | 0.5 |
| Monobasic sodium phosphate | 0.05 |
| Dibasic sodium phosphate (anhydrous) | 0.15 |
| Sodium chloride | 0.75 |
| Disodium EDTA (Edetate disodium) | 0.05 |
| Benzalkonium chloride | 0.01 |
| NaOH and/or HCl | pH 7.3 - 7.4 |
| Purified water | q.s. to 100% |

| FORMULATION 4 | |
|---|---|
| **Ingredient** | **Amount (wt%)** |
| Fluprostenol isopropyl ester (Table 2, Compound **B**) | 0.003 |
| Monobasic sodium phosphate | 0.05 |
| Dibasic sodium phosphate (anhydrous) | 0.15 |
| Sodium chloride | 0.75 |
| Disodium EDTA (Edetate disodium) | 0.05 |
| Benzalkonium chloride | 0.01 |
| HCl and/or NaOH | pH 7.3 - 7.4 |
| Purified water | q.s. to 100% |

| FORMULATION 5 | |
|---|---|
| **Ingredient** | **Amount (wt%)** |
| Compound **5** (Table 1) | 0.002 |
| Dextran 70 | 0.1 |
| Hydroxypropyl methylcellulose | 0.3 |
| Sodium chloride | 0.77 |
| Potassium chloride | 0.12 |
| Disodium EDTA | 0.05 |
| Benzalkonium chloride | 0.01 |
| HCl and/or NaOH | pH 7.2 - 7.5 |
| Purified water | q.s. to 100% |

| FORMULATION 6 | |
|---|---|
| **Ingredient** | **Amount (wt%)** |
| Compound **6** (Table 1) | 0.01 |
| Monobasic sodium phosphate | 0.05 |
| Dibasic sodium phosphate (anhydrous) | 0.15 |
| Sodium chloride | 0.75 |
| Disodium EDTA | 0.01 |
| Benzalkonium chloride | 0.02 |
| Polysorbate 80 | 0.15 |
| HCl and/or NaOH | pH 7.3 - 7.4 |
| Purified water | q.s. to 100% |

| FORMULATION 7 | |
|---|---|
| **Ingredient** | **Amount (wt%)** |
| Compound **7** (Table 1) | 0.001 |
| Dextran 70 | 0.1 |
| Hydroxypropyl methylcellulose | 0.5 |
| Monobasic sodium phosphate | 0.05 |
| Dibasic sodium phosphate (anhydrous) | 0.15 |
| Sodium chloride | 0.75 |
| Disodium EDTA | 0.05 |
| Benzalkonium chloride | 0.01 |
| NaOH and/or HCl | pH 7.3 - 7.4 |
| Purified water | q.s. to 100% |

| FORMULATION 8 | |
|---|---|
| **Ingredient** | **Amount (wt%)** |
| Compound **8** (Table 1) | 0.003 |
| Monobasic sodium phosphate | 0.05 |
| Dibasic sodium phosphate (anhydrous) | 0.15 |
| Sodium chloride | 0.75 |
| Disodium EDTA | 0.05 |
| Benzalkonium chloride | 0.01 |
| HCl and/or NaOH | pH 7.3 - 7.4 |
| Purified water | q.s. to 100% |

## Claims

1. Use of fluprostenol isopropyl ester as the sole active ingredient for the manufacture of a medicament for topical application for the treatment of glaucoma and ocular hypertension.

## Patentansprüche

1. Verwendung von Fluprostenolisopropylester als das einzige aktive Ingredienz für die Herstellung eines Medikaments zur topischen Anwendung für die Behandlung von Glaukom und okularer Hypertension.

## Revendications

1. Utilisation de l'ester isopropylique de fluprosténol à titre d'ingrédient actif unique pour la préparation d'un médicament destiné à une application locale pour le traitement du glaucome et de l'hypertension oculaire.
